# EUROPÄISCHE PATENTSCHRIFT

(11) **EP 1 202 702 B1**
(45) Veröffentlichungstag und Bekanntmachung des Hinweises auf die Patenterteilung: **26.11.2003**
(21) Anmeldenummer: 00965739.6
(22) Anmeldetag: 10.08.2000
(51) Int. Cl.: A61K 6/02, A61L 27/28

(54) **WERKSTÜCK UND VERFAHREN ZUM HERSTELLEN SOWIE ZUM VERWERTEN DIESES WERKSTÜCKS**
WORK PIECE AND METHOD FOR PRODUCING AND UTILIZING SAID WORK PIECE
PIECE ET PROCEDE POUR PRODUIRE ET UTILISER CETTE DERNIERE

(30) Priorität: 13.08.1999 DE 19937864
(43) Veröffentlichungstag der Anmeldung: 08.05.2002
(73) Patentinhaber: Marx, Rudolf, 53533 Eichenbach (DE); Fischer, Horst, 52074 Aachen (DE)
(72) Erfinder: Marx, Rudolf, 53533 Eichenbach (DE); Fischer, Horst, 52074 Aachen (DE)
(74) Vertreter: König, Werner, Dipl.-Ing.
(86) Internationale Anmeldenummer: DE0002702
(87) Internationale Veröffentlichungsnummer: WO01012131

(56) Entgegenhaltungen:
- EP-A- 0 434 010
- WO-A-94/15652
- US-A- 4 364 731
- US-A- 4 478 579
- US-A- 5 437 937

## Beschreibung

Die Erfindung betrifft ein Werkstück mit einem Substrat aus Keramik, Metall oder Polymer, wobei das Substrat eine zur Bildung einer stabilen Verbindung mit einem Polymer konditionierte Oberfläche hat, die mit einer Siliziumoxidschicht und darüber mit einem Silanhaftvermittler versehen ist. Femer betrifft die Erfindung Verfahren zum Herstellen und Verwerten des erfindungsgemäßen Werkstückes.

US-A-5 437 937 offenbart ein Verfabren zur Verbesserung der Adhäsionseigenschaften einer metallischen Oberfläche.

Es ist aus US-A-4364731 für die Herstellung von Zahnkronen bekannt, auf eine gereinigte keramische, metallische oder polymere Substratoberfläche eine Siliziumoxidschicht aufzusputtern, auf diese einen Silanhaftvermittler und darauf abschließend eine polymere Endschicht ("Kunststoffverblendung") aufzubringen. Ein derartiger Beschichtungsvorgang muss ortsgebunden innerhalb kürzester Zeit abgeschlossen sein, da ansonsten bei offenliegender Silanhaftvermittlerschicht eine physikalische oder chemische Beschädigung der äußerst empfindlichen Oberfläche, z.B. durch Abrieb, eine makroskopische Verunreinigung, z.B. in Form von Hautschuppen, oder eine chemische Kontamination der reaktiven Schichtoberfläche, z.B. durch Reaktion mit Stickstoff, Kohlenwasserstoffen oder anderen Luftbestandteilen, eintreten kann. Eine Lagerung und ein Transport der allein mit Siliziumoxid beschichteten und mit Silanhaftvermittler versehenen Werkstücke, wobei die polymere Endbeschichtung erst später und/oder an einem anderen Ort stattfindet, ist nach diesem Verfahren nicht durchführbar.

An eine derartig verblendete Zahnkrone wird für die zahnärztliche Routine keineswegs die Forderung nach Sterilität im Sinne von Keimfreiheit gestellt. Sie wird üblicherweise lediglich grob mit Ethanol gereinigt. Die Forderung nach Sterilität einer Zahnkrone wäre aber auch praxisfern, da eine Zahnkrone nach der Herstellung durch den Zahntechniker ohne besondere Forderungen an die Sterilität verpackt wird. Sie wird nach der Ankunft beim Zahnarzt durch diesen und seine Helferin vor der Eingliederung zwar unter Beachtung gewisser Hygienevorschriften aber auch durch Berühren mit den Fingern gehandhabt. Die Art der Handhabung schließt also den Fortbestand einer eventuell vorhandenen Sterilität aus. Spätestens bei der Eingliederung in den Mund wird die Krone sofort massiv von Keimen und anderen mundtypischen Mikroorganismen besiedelt, so dass eine vorherige Sterilität gegenstandslos würde. Auch die gesunde Mundhöhle ist nämlich "physiologisch" stets stark von Keimen besiedelt. Diese Flora ist für die Verdauung von Wichtigkeit, da der Verdauungsprozess initial durch den Speichel im Mund beginnt.

Auch wirkt bei einer derartigen Zahnkrone eine Polymerbeschichtung keineswegs als Schutzschicht vor physikalisch-mechanischen oder sonstigen Belastungen. Es handelt sich vielmehr um eine ästhetische Verblendung (typische Dicke 1 mm) für eine zumeist metallische, gegebenenfalls auch keramische oder polymere Zahnkrone. Mit der Verblendung durch den Kunststoff ist keine Schutzfunktion für die Kronenbasis beabsichtigt. Es liegt z. B. keine Schutzfunktion vor gegen:
a) physikalischen Abrieb: Kaufunktion (die Kronenbasis selber ist wesentlich abriebfester als die polymere Verblendung), oder
b) chemische Einflüsse: korrosiver Angriff durch Speichel-, Nahrungsmittel- und Medikamenteneinfluss, mikrobielle Effekte, z.B. durch Säureausscheidungen von Bakterien (die Kronenbasis, insbesondere bei Verwendung von Edelmetallen oder Keramik, ist wesentlich korrosionsstabiler als die Kunststoffbeschichtung), oder
c) Temperaturwechseleinflüsse bei der Nahrungsaufnahme: sie können durch unterschiedliche Ausdehnungskoeffizienten zyklisch wechselnde Spannungen bedingen, die die mechanische Stabilität beeinträchtigen (die Kronenbasis ist wesentlich zyklenfester als die Verblendung).

Es ist zu beachten, dass die Kronenbasis in der Regel (in Deutschland) aus einer hochgoldhaltigen Legierung besteht. Üblich sind auch Ni- und CoCr-Legierungen (aus der Rüstungstechnik auch als "Superalloys" bekannt), ferner Pd-Basislegierungen (nur eingeschränkt biokompatibel; "Sparlegierungen"; diese sind umstritten, da sie ein hohes Allergiepotential aufweisen) oder unlegiertes Titan (abgesehen von der Farbe und der schwierigen Verarbeitbarkeit unter den obigen Gesichtspunkten den Goldlegierungen ebenbürtig). Alle Legierungen sind auf Mundstabilität ausgelegt und diese ist insbesondere bei hochgoldhaltigen Legierungen gewährleistet. Die Kunststoffverblendung als eine die Ästhetik verbessernde Maßnahme unterstützt die Mundstabilität nicht zusätzlich; dies ist auch nicht beabsichtigt. Kunststoffe sind nämlich aufgrund ihrer materialkundlichen Eigenschaften prinzipiell weniger mundstabil als hochkorrosionsfeste Metalle oder Keramiken.

Die Kunststoffverblendungen kommen in erster Linie für den temporären und den herausnehmbaren Zahnersatz (Reparaturmöglichkeit, da hoher Abrieb und Abplatzen des Kunststoffes) in Betracht.

Das Produkt Zahnkrone mit dem Schichtsystem "Substrat, Silikat-, Silanschicht, Kunststoffverblendung" stellt also ein fertiges Endprodukt dar, das in dieser Form beim Patienten eingesetzt werden kann.

Die Aufgabe der vorliegenden Erfindung besteht nun darin, ein Werkstück in Form eines Halbzeuges, insbesondere eines Halbzeugimplantates bzw. einer Halbzeugprothese, mit einer guten Verbundfestigkeit zu mindestens einer noch aufzubringenden Komponente eines Mehrkomponentenadhäsivs zu schaffen, wobei das Halbzeug bis zu dem zeitlich versetzten Aufbringen der Adhäsivkomponente eine steril konservierende Oberfläche aufweisen soll, die zur Lagerung und auch zum Transport geeignet ist.

Des weiteren sind Verfahren zum Herstellen und Verwerten des erfindungsgemäßen Werkstückes vorgesehen.

Zur Lösung dieser Aufgabe ist erfindungsgemäß bei einem Werkstück der eingangs erwähnten Art vorgesehen, dass das Substrat, die Siliziumoxidschicht und der Silanhaftvermittler steril sind und dass sich auf dem Silanhaftvermittler eine sterile und/oder nach der Auspolymerisation sterilisierbare konservierende Schutzschicht als aktivierbare erste Komponente eines Mehrkomponentenadhäsivs befindet, das sich im Einsatz durch Zugabe mindestens einer weiteren Adhäsivkomponente bildet.

Für den Schichtaufbau zwischen Substrat und Schutzschicht bzw. Mehrkomponentenadhäsiv ist Keimfreiheit im Gegensatz zur Zahnkrone unabdingbar vonnöten, da - wenn das Schichtsystem des Halbzeugs fertiggestellt ist- gegebenenfalls im Schichtsystem befindliche Keime nicht mehr entfernt werden können. Nur mit einem derartig präparierten Schichtsystem kann ein reibungsloser Einsatz, z. B. als Hüftoder Knieprothese, garantiert werden. Bei einem nicht keimfreien Schichtaufbau besteht die Gefahr von Infektionen im Körper, die unbedingt vermieden werden müssen. Derartige Infektionen sind nur schwer zu bekämpfen, zumal die Widerstandskräfte des Patienten aufgrund des aufwendigen operativen Eingriffs geschwächt sind. Gegebenenfalls müsste als Folge der Infektion die erste Hüftendoprothese in einem erheblichen operativen Aufwand wieder entnommen, die Infektion bekämpft und eine zweite Hüftendoprothese eingesetzt werden. Hierbei muss darauf hingewiesen werden, dass pro Patient eine derartige Hüftoperation nur maximal drei- bis viermal durchgeführt werden kann.

Durch die konservierende Schutzschicht wird der Silanhaftvermittler chemisch abgebunden und kann folglich nicht mehr physikalisch oder chemisch kontaminiert werden. Die Schichtdicke der Schutzschicht ist zudem ausreichend, um einer geringen Reibung standzuhalten. Dadurch wird ein lager- und transportierbares Werkstück geschaffen. Es können so größere Anzahlen von Halbzeugen an einem Produktionsstandort hergestellt und später an einem anderen Standort mit der/den weiteren Adhäsivkomponenten versehen werden. Da das erfindungsgemäße Halbzeug an wenigen zentralen Hightech-Produktionsstätten angefertigt und dann an die Verbraucher, z. B. Krankenhäuser im In- und Ausland, versendet werden soll, kann es zu Lagerungszeiten von einigen Monaten kommen, bevor die Werkstücke operativ eingesetzt werden können. Über diesen gesamten Zeitraum, der durchaus sechs bis zwölf Monate umspannen kann, muss die Sterilität des Werkstückes garantiert werden.

Im Einsatz kann die Schutzschicht, die keine Endschicht, sondern eine Adhäsivkomponente eines Mehrkomponentenadhäsivs ist, vor Ort mit einer zweiten Adhäsivkomponente, z. B. einem sterilen MMA-Monomer, bestrichen und dadurch aktiviert werden. Direkt im Anschluss werden diese beiden Komponenten dann mit einer dritten Adhäsivkomponente, einem polymeren Adhäsiv, in Verbindung gebracht.

Erfindungsgemäß ist ferner vorgesehen, dass die sterile und/oder sterilisierbare konservierende Schutzschicht aus Polymethylmethacrylat hergestellt sein kann. Eine solche Schutzschicht ist besonders in der Medizin zweckmäßig, wenn das Werkstück als Implantat in einem Knochen festzusetzen ist.

Erfindungsgemäß ist ferner vorgesehen, dass die sterile und/oder sterilisierbare konservierende Schutzschicht aus BisGMA hergestellt sein kann. Eine Schutzschicht aus BisGMA ist besonders in der Zahnmedizin anzuwenden.

Erfindungsgemäß ist ferner vorgesehen, dass die konservierende Schutzschicht aus Epoxidharz hergestellt sein kann. Eine Schutzschicht aus Epoxidharz ist besonders im technischen, nicht-biologischen Bereich anzuwenden.

Erfindungsgemäß ist ferner vorgesehen, dass die konservierende Schutzschicht aus Phenolharz hergestellt sein kann. Eine Schutzschicht aus Phenolharz ist besonders im technischen, nicht-biologischen Bereich anzuwenden.

Erfindungsgemäß ist ferner vorgesehen, dass die sterile und/oder sterilisierbare, konservierende Schutzschicht <100 µm dick sein kann. Eine derart dünne Schutzschicht kann sehr schnell durch die Benetzung mit einem entsprechenden Monomer aktiviert werden. Diese Schicht ist zudem ausreichend dick gewählt, um beim Transport einen Schutz gegen Abrieb zu bilden.

Erfindungsgemäß ist ferner vorgesehen, dass das Substrat eine zur Bildung einer stabilen Verbindung mit einem polymeren Adhäsiv konditionierte Oberfläche haben kann.

Erfindungsgemäß ist ferner vorgesehen, dass das Werkstück in feucht-warmen Medien verwendet werden kann.

Erfindungsgemäß ist ferner vorgesehen, dass das Werkstück als Implantat, Prothese oder als Komponente von Implantat bzw. Prothese in der Medizin verwendet werden kann. Hierbei sind insbesondere Implantate in Form von sterilen und/oder sterilisierbaren, beschichteten Hüftendoprothesenschäften, Kniegelenksprothesenschäften und sonstigen Gelenksprothesenschäften vorgesehen.

Erfindungsgemäß ist femer ein Verfahren zum Herstellen eines obigen Werkstückes vorgesehen, bei dem die Oberfläche des Substrates gereinigt wird, dann eine Siliziumoxidschicht unter Verwendung einer Hochvakuumverdampfungsanlage aufgebracht und nachfolgend mit einem Silanhaftvermittler benetzt wird, wobei nach der Reinigung der Substratoberfläche auf dieser durch ein Niederdruckplasmaverfahren Carboxylgruppen erzeugt werden und auf die Siliziumoxidschicht und den Silanhaftvermittler zur Konservierung der derart behandelten Oberfläche bis zur Weiterverarbeitung eine sterile und/oder sterilisierbare konservierende Schutzschicht als aktivierbare erste Komponente eines Mehrkomponentenadhäsivs aufgetragen wird, das sich im Einsatz durch Zugabe mindestens einer weiteren Adhäsivkomponente bildet. Dabei wird die üblicherweise bereits vorgereinigte Substratoberfläche durch das Niederdruckplasmaverfahren einer weiteren Reinigung unterzogen. Durch Auswahl eines geeigneten Plasmagases können Carboxylgruppen auf der gereinigten Substratoberfläche zur Aktivierung erzeugt werden, was eine deutliche Heraufsetzung der Haftfestigkeit bewirkt. Durch das Aufbringen einer sterilen und/oder sterilisierbaren konservierenden Schutzschicht sind lager- und transportierbare Werkstücke herstellbar.

Erfindungsgemäß ist ferner vorgesehen, dass das Aufdampfen der Siliziumoxidschicht reproduzierbar mittels eines Shuttersystems erfolgen kann. Es kann zusätzlich eine Dreiachsenverfahreinheit vorgesehen sein, mit der auch auf einer komplexen 3D-Oberfläche eine Siliziumoxidschicht konstanter Dicke realisierbar wird.

Erfindungsgemäß ist ferner ein Verfahren zum Verwerten eines obigen Werkstückes vorgesehen, wobei das Werkstück nach steriler Zwischenlagerung auf seiner konditionierten Oberfläche zunächst zur Aktivierung der Schutzschicht mit einer monomeren Adhäsivkomponente versehen wird und darüber eine polymere Adhäsivkomponente aufgebracht wird, wobei diese beiden Adhäsivkomponenten mit der Schutzschicht ein Mehrkomponentenadhäsiv bilden. Durch die Aktivierung der konservierenden Schutzschicht mit der monomeren Adhäsivkomponente kann die Schutzschicht mit der aufzubringenden polymeren Adhäsivkomponentenschicht chemisch reagieren. Durch die sterile Aufbewahrung des Werkstückes, z. B. unter He- oder N₂-Schutzgas in einer gasdichten Blisterpackung, ist eine mehrmonatige Zwischenlagerung bis zur Weiterverarbeitung möglich.

Das erfindungsgemäße Werkstück und die Verfahren zum Herstellen und Verwerten desselben werden im folgenden anhand eines Hüftendoprothesenschaftes dargestellt:
Auf einem gereinigten metallischen Schaft einer Hüftendoprothese werden mittels eines Niederdruckplasmaverfahrens Carboxylgruppen erzeugt, welche die Haftfestigkeit einer darauf aufzudampfenden Siliziumoxidschicht erhöhen. Diese Siliziumoxidschicht wird vorzugsweise monomolekular mit einem Silanhaftvermittler benetzt, und auf diesen wird eine sterile und/oder sterilisierbare konservierende Schutzschicht aufgetragen.
Die derart hergestellte Hüftendoprothese weist folglich einen metallischen Schaft mit einer Siliziumoxidschicht, einer darüberliegenden Schicht aus Silanhaftvermittler und einer abschließenden sterilen und/oder sterilisierten konservierenden Schicht (erste Adhäsivkomponente) auf.

Nach der Herstellung wird die erfindungsgemäße Hüftendoprothese steril verpackt, z. B. in einer mit He- oder N₂-Schutzgas befüllten, gasdichten Blisterverpackung. Dadurch ist sie vor Verschmutzung geschützt und kann ohne Qualitätsverluste sowohl transportiert als auch über mehrere Monate gelagert werden. Der beschichtete Schaft der Hüftendoprothese wird der sterilen Verpackung anschließend vor Ort unter sterilen Bedingungen im Operationssaal entnommen und mit einem sterilen MMA-Monomer (weitere Adhäsivkomponente) bestrichen. Dadurch wird die sterile konservierende Schutzschicht aktiviert. Direkt im Anschluß wird der Schaft in eine mit dem polymerem Adhäsiv (Knochenzement = weitere Adhäsivkomponente) gefüllte Knochenbohrung versenkt. Nach Verfestigung des Adhäsivs ist zwischen dem Adhäsiv und dem Schaft der Hüftendoprothese eine Verbindung hoher und unter den feucht-warmen Bedingungen des Körpers langzeitstabile Haftfestigkeit entstanden.

## Patentansprüche

1. Werkstück mit einem Substrat aus Keramik, Metall oder Polymer, wobei das Substrat eine zur Bildung einer stabilen Verbindung mit einem Polymer konditionierte Oberfläche hat, die mit einer Siliziumoxidschicht und darüber mit einem Silanhaftvermittler versehen ist,
**dadurch gekennzeichnet,**
- **dass** das Substrat, die Siliziumoxidschicht und der Silanhaftvermittler steril sind und
- **dass** auf dem Silanhaftvermittler eine sterile und/oder nach der Auspolymerisation sterilisierbare konservierende Schutzschicht als aktivierbare erste Komponente eines Mehrkomponentenadhäsivs vorgesehen ist, das sich im Einsatz durch Zugabe mindestens einer weiteren Adhäsivkomponente bildet.

2. Werkstück nach Anspruch 1, **dadurch gekennzeichnet, dass** die sterile und/oder sterilisierbare konservierende Schutzschicht aus Polymethylmethacrylat hergestellt ist.

3. Werkstück nach Anspruch 1, **dadurch gekennzeichnet, dass** die sterile und/oder sterilisierbare konservierende Schutzschicht aus BisGMA hergestellt ist.

4. Werkstück nach Anspruch 1, **dadurch gekennzeichnet, dass** die konservierende Schutzschicht aus Epoxidharz hergestellt ist.

5. Werkstück nach Anspruch 1, **dadurch gekennzeichnet, dass** die konservierende Schutzschicht aus Phenolharz hergestellt ist.

6. Werkstück nach einem der vorhergehenden Ansprüche, **dadurch gekennzeichnet, dass** die sterile und/oder sterilisierbare konservierende Schutzschicht <100 µm dick ist.

7. Werkstück nach einem der vorhergehenden Ansprüche, **dadurch gekennzeichnet, dass** das Substrat eine zur Bildung einer stabilen Verbindung mit einem polymeren Adhäsiv konditionierte Oberfläche hat.

8. Werkstück nach einem der vorhergehenden Ansprüche, **dadurch gekennzeichnet, dass** es in feucht-warmen Medien verwendet wird.

9. Werkstück nach einem der vorhergehenden Ansprüche, **dadurch gekennzeichnet, dass** es als Implantat, Prothese oder als Komponente von Implantat bzw. Prothese in der Medizin verwendet wird.

10. Verfahren zum Herstellen eines Werkstückes nach einem der vorherigen Ansprüche, bei dem die Oberfläche des Substrates gereinigt wird, dann eine Siliziumoxidschicht unter Verwendung einer Hochvakuumverdampfungsanlage aufgebracht und nachfolgend mit einem Silanhaftvermittler benetzt wird,
**dadurch gekennzeichnet,**
**dass** nach der Reinigung der Substratoberfläche auf dieser durch ein Niederdruckplasmaverfahren Carboxylgruppen erzeugt werden und
**dass** auf die Siliziumoxidschicht und den Silanhaftvermittler zur Konservierung der derart behandelten Oberfläche bis zur Weiterverarbeitung eine sterile und/oder sterilisierbare konservierende Schutzschicht, als aktivierbare erste Komponente eines Mehrkomponentenadhäsivs aufgetragen wird, das sich im Einsatz durch Zugabe mindestens einer weiteren Adhäsivkomponente bildet.

11. Verfahren zum Herstellen eines Werkstückes nach Anspruch 10, **dadurch gekennzeichnet, dass** das Aufdampfen der Siliziumoxidschicht reproduzierbar mittels eines Shuttersystems erfolgt.

12. Verfahren zum Verwerten eines Werkstückes nach einem der vorherigen Ansprüche,
**dadurch gekennzeichnet,**
**dass** das Werkstück nach steriler Zwischenlagerung auf seiner konditionierten Oberfläche zunächst zur Aktivierung der Schutzschicht mit einer monomeren Adhäsivkomponente versehen wird und darüber eine polymere Adhäsivkomponente aufgebracht wird, wobei diese beiden Adhäsivkomponenten mit der Schutzschicht ein Mehrkomponentenadhäsiv bilden.

## Claims

1. Workpiece with a substrate of ceramic, metal or polymer, said substrate having a surface which is conditioned to form a stable connection to a polymer and is provided with a silicon oxide layer and, over the latter, with a silane bonding agent,
**characterized in that**
- the substrate, the silicon oxide layer and the silane bonding agent are sterile, and
- on top of the silane bonding agent, a preserving protective layer which is sterile and/or sterilizable after polymerization is provided as the activatable first component of a multi-component adhesive which at the time of use is formed by addition of at least one further adhesive component.

2. Workpiece according to Claim 1, **characterized in that** the sterile and/or sterilizable preserving protective layer is produced from polymethyl methacrylate.

3. Workpiece according to Claim 1, **characterized in that** the sterile and/or sterilizable preserving protective layer is produced from BisGMA.

4. Workpiece according to Claim 1, **characterized in that** the preserving protective layer is produced from epoxy resin.

5. Workpiece according to Claim 1, **characterized in that** the preserving protective layer is produced from phenolic resin.

6. Workpiece according to one of the preceding claims, **characterized in that** the sterile and/or sterilizable preserving protective layer has a thickness of < 100 µm.

7. Workpiece according to one of the preceding claims, **characterized in that** the substrate has a surface which is conditioned to form a stable connection to a polymeric adhesive.

8. Workpiece according to one of the preceding claims, **characterized in that** it is used in moist warm media.

9. Workpiece according to one of the preceding claims, **characterized in that** it is used as an implant or prosthesis or as a component of an implant or prosthesis in medicine.

10. Method for producing a workpiece according to one of the preceding claims, in which method the surface of the substrate is cleaned, a silicon oxide layer is then applied using a high-vacuum evaporation unit and is then wetted with a silane bonding agent,
**characterized in that**
after the substrate surface has been cleaned, carboxyl groups are generated thereon by means of a low-pressure plasma process, and
a sterile and/or sterilizable preserving protective layer is applied to the silicon oxide layer and to the silane bonding agent in order to preserve the surface thus treated until further processing, said sterile and/or sterilizable preserving protective layer being applied as the activatable first component of a multi-component adhesive which at the time of use is formed by addition of at least one further adhesive component.

11. Method for producing a workpiece according to Claim 10, **characterized in that** the vapour-deposition of the silicon oxide layer is effected in a reproducible manner using a shutter system.

12. Method for utilizing a workpiece according to one of the preceding claims,
**characterized in that**
after sterile intermediate storage, the workpiece is first provided on its conditioned surface with a monomeric adhesive component in order to activate the protective layer, and a polymeric adhesive component is then applied on top of this, these two adhesive components forming a multi-component adhesive together with the protective layer.

## Revendications

1. Pièce d'oeuvre avec un substrat en céramique, métal ou polymère, où le substrat comporte une surface conditionnée pour établir une liaison solide avec un polymère, laquelle surface est pourvue d'une couche d'oxyde de silicium et d'un agent adhésif de silane par-dessus,
**caractérisée en ce que** :
- le substrat, la couche d'oxyde de silicium et l'agent adhésif de silane sont stériles et,
- sur l'agent adhésif de silane est prévue une couche de protection stérile et/ou stérilisable et de conservation après la fin de la polymérisation en guise de premier composant activable d'un adhésif à plusieurs composants, qui se forme lors de l'utilisation en rajoutant au moins un autre composant adhésif.

2. Pièce d'oeuvre selon la revendication 1, **caractérisée en ce que** la couche de protection stérile et/ou stérilisable et de conservation est fabriquée en polyméthacrylate de méthyle.

3. Pièce d'oeuvre selon la revendication 1, **caractérisée en ce que** la couche de protection stérile et/ou stérilisable et de conservation est fabriquée en BisGMA.

4. Pièce d'oeuvre selon la revendication 1, **caractérisée en ce que** la couche de protection et de conservation est fabriquée en résine époxy.

5. Pièce d'oeuvre selon la revendication 1, **caractérisée en ce que** la couche de protection et de conservation est fabriquée en résine phénolique.

6. Pièce d'oeuvre selon l'une quelconque des revendications précédentes, **caractérisée en ce que** la couche de protection stérile et/ou stérilisable et de conservation est d'une épaisseur < 100 µm.

7. Pièce d'oeuvre selon l'une quelconque des revendications précédentes, **caractérisée en ce que** le substrat possède une surface conditionnée pour former une liaison solide avec un adhésif polymère.

8. Pièce d'oeuvre selon l'une quelconque des revendications précédentes, **caractérisée en ce qu'**elle est utilisée dans des milieux chauds et humides.

9. Pièce d'oeuvre selon l'une quelconque des revendications précédentes, **caractérisée en ce qu'**elle est utilisée en tant qu'implant, prothèse ou composant d'un implant et/ou d'une prothèse, en médecine.

10. Procédé pour la fabrication d'une pièce d'oeuvre selon l'une quelconque des revendications précédentes, au cours duquel la surface du substrat est nettoyée, une couche d'oxyde de silicium est ensuite appliquée en utilisant un dispositif d'évaporation sous vide poussé, et est ensuite imprégnée avec un agent adhésif de silane,
**caractérisé en ce**
**qu'**après le nettoyage de la surface du substrat, des groupes carboxyles sont produits sur celle-ci à l'aide d'un procédé au plasma basse pression, et en ce qu'une couche de protection stérile et/ou de conservation est ensuite appliquée sur la couche d'oxyde de silicium et l'agent adhésif de silane pour la conservation de la surface ainsi traitée jusqu'au traitement ultérieur en guise de premier composant activable d'un adhésif à plusieurs composants, qui se forme lors de l'utilisation en rajoutant au moins un autre composant adhésif.

11. Procédé pour la fabrication d'une pièce d'oeuvre selon la revendication 10, **caractérisé en ce que** la métallisation sous vide de la couche d'oxyde de silicium s'effectue de manière reproductible moyennant un système d'obturation.

12. Procédé pour l'utilisation d'une pièce d'oeuvre selon l'une quelconque des revendications précédentes,
**caractérisé en ce que**
la pièce d'oeuvre, après un entreposage intermédiaire stérile sur sa surface conditionnée, est tout d'abord pourvue d'un composant adhésif monomère pour l'activation de la couche de protection, et **en ce qu'**un composant adhésif polymère est appliqué par-dessus, moyennant quoi ces deux composants adhésifs forment, avec la couche de protection, un adhésif à plusieurs composants.
